(19) Europäisches Patentamt
European Patent Office
Office européen des brevets

(11) Veröffentlichungsnummer: **0 206 995**
**B1**

# (12) EUROPÄISCHE PATENTSCHRIFT

(45) Veröffentlichungstag der Patentschrift: **24.01.90**

(51) Int. Cl.$^5$: **C 07 D411/12**, A 01 N 47/36

(21) Anmeldenummer: **86810263.3**

(22) Anmeldetag: **12.06.86**

(54) **N-Heterocyclosulfonyl-N'-pyrimidinyl-, N'-triazolyl- und N'-triazinyl-harnstoffe.**

(30) Priorität: **18.06.85 CH 2566/85**
**05.03.86 CH 894/86**

(43) Veröffentlichungstag der Anmeldung: **30.12.86 Patentblatt 86/52**

(45) Bekanntmachung des Hinweises auf die Patenterteilung: **24.01.90 Patentblatt 90/04**

(84) Benannte Vertragsstaaten: **AT BE CH DE FR GB IT LI NL**

(56) Entgegenhaltungen:
**EP-A- 0 099 339**
**EP-A- 0 107 979**
**EP-A- 0 132 230**
**EP-A- 0 134 477**
**EP-A- 0 168 135**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Föry, Werner, Dr.**
**Inzlingerstrasse 11**
**CH-4125 Riehen (CH)**
Erfinder: **Meyer, Willy**
**Talweg 49**
**CH-4125 Riehen (CH)**

**EP 0 206 995 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die vorliegende Erfindung betrifft neue, herbizid wirksame und pflanzenwuchsregulierende N-(3,4-Dihydro-2,2-dioxo-1,2-benzoxathiin-8-ylsulfonyl)-N'-pyrimidinyl, N'-triazolyl- oder N'-triazinyl-harnstoffe, Verfahren zu ihrer Herstellung, die sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums.

Die erfindungsgemässen Wirkstoffe entsprechen der Formel I

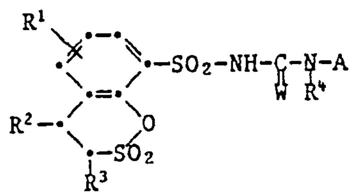

(I)

worin

$R^1$ Wasserstoff, Halogen, Nitro, $C^1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_5$-Alkoxyalkoxy,

$R^2$ und $R^3$ unabhängig voneinander Wasserstoff oder $C_1$-$C_4$-Alkyl,

$R^4$ Wasserstoff, Methyl oder Aethyl,

W Sauerstoff oder Schwefel, und

A einen Rest

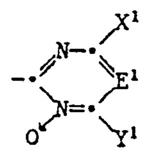

,

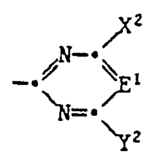

,

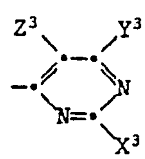

,

A1 A2 A3

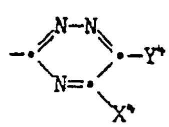

,

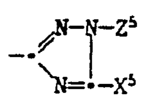

,

A4 A5

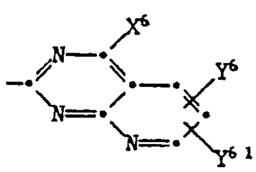

,

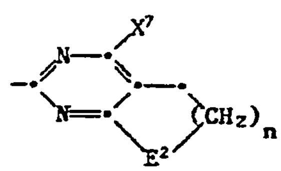

oder

A6 A7

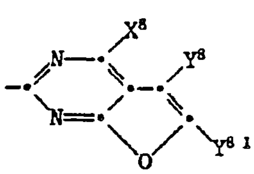

A8

bedeuten, wobei

$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halogenalkyl, $C_1$-$C_4$-Alkoxy, $C_1$-$C_4$-Alkyl, Dimethylamino, Methylamino, Aethylamino, Amino oder $C_2$-$C_4$-Alkoxyalkyl,

$E^1$ für Stickstoff oder die Methinbrücke,

$E^2$ für Sauerstoff oder die Methylenbrücke,

$Y^2$ für Cyclopropyl, Dimethoxymethyl, Diäthoxymethyl

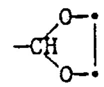

oder

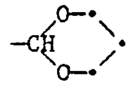

$Y^3$ für Methyl, Methoxy, Aethyl, Aethoxy, $C_1$-$C_2$-Halogenalkyl oder $C_1$-$C_2$-Halogenalkoxy,
$Y^4$ für Wasserstoff, Methoxy, Aethoxy, Halogen oder $C_1$-$C_4$-Alkyl,
$Y^6$ und $Y^{6\ 1}$ unabhängig voneinander für Wasserstoff, $C_1$-$C_4$-Alkyl, Halogen, Methoxy oder Methylthio,
$Y^8$ und $Y^{8\ 1}$ unabhängig voneinander für Wasserstoff oder Methyl,
n für eins oder zwei,
$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,
$Z^3$ und $Y^3$ zusammen für eine $C_2$-$C_4$-Alkylenbrücke oder eine durch Sauerstoff unterbrochene $C_2$-$C_4$-Alkylenbrücke, und
$Z^5$ für Methyl oder Aethyl stehen,
sowie den Salzen dieser Verdindungen ; mit der Massgabe, dass $R^3$ $C_1$-$C_4$-Alkyl bedeutet wenn A für einen der Reste A2, A5, A7 oder A8 steht.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den veröffentlichten europäischen Patentanmeldungen 99 339, 107 979 und 168 135 beschrieben.

In den Definitionen ist unter Alkyl geradkettiges oder verzweigtes Alkyl zu verstehen ; z. B. : Methyl, Aethyl, n-Propyl, i-Propyl oder die vier isomeren Butyl.

Unter Alkoxy ist zu verstehen : Methoxy, Aethoxy, n-Propyloxy, i-Propyloxy oder die vier isomeren Butyloxy, insbesondere aber Methoxy, Aethoxy oder i-Propyloxy.

Beispiele für Alkylthio sind Methylthio, Aethylthio, n-Propylthio, i-Propylthio oder die vier isomeren Butylthio, insbesondere aber Methylthio und Aethylthio.

Unter Halogen selbst sowie als Teil eines Substituenten wie in Halogenalkoxy, Halogenalkylthio oder Halogenalkyl sind Fluor, Chlor und Brom, vorzugsweise jedoch Fluor und Chlor zu verstehen. Halogenalkyl selbst oder als Teil von Halogenalkoxy oder Halogenalkylthio steht in der Regel für Chlormethyl, Fluormethyl, Difluormethyl, Trifluormethyl, 2-Chloräthyl, 2,2,-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Beispiele für Alkoxyalkyl sind : Methoxymethyl, Methoxyäthyl, Methoxypropyl, Aethoxyäthyl, Aethoxymethyl oder Propyloxymethyl. Beispiele für Alkoxyalkoxy sind : Methoxymethoxy, Methoxyäthoxy, Methoxypropyloxy, Aethoxymethoxy, Aethoxyäthoxy sowie Propyloxymethoxy. Alkylengruppen sind im Rahmen der Definition unter Formel I Aethylen, Propylen, Butylen, 1-Methyläthylen, 1-Aethyläthylen, 2-Methylbutylen, 1-Methylbutylen oder wenn die Brücke durch Sauerstoff unterbrochen ist auch $-CH_2-O-CH_2-$ oder $-CH_2-O-CH_2-CH_2-$.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Träthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin, Diäthanolamin und 1,4-Diazabicyclo[2.2.2]octan.

Beispiele für quaternäre Ammoniumbasen sind im allgemeinen die Kationen von Halogenammoniumsalzen, z. B. das Tetramethylammoniumkation, das Tetraäthylammoniumkation, das Trimethyläthylammoniumkation, aber auch das Ammoniumkation.

Unter den Verbindungen der Formel I sind diejenigen bevorzugt, in denen entweder

a) W Sauerstoff ist oder
b) $R^1$ und $R^2$ für Wasserstoff, und $R^3$ für Methyl stehen oder
c) $R^4$ für Wasserstoff steht.

Als weitere hervorzuhebende Untergruppe von Verbindunger der Formel I ist diejenige zu nennen, in denen W für Sauerstoff und $R^4$ für Wasserstoff stehen.

Besonders bevorzugte Untergruppen von Verbindungen der Formel I sind dadurch charakterisiert,

dass entweder

aa) W für Sauerstoff, $R^4$ für Wasserstoff und A für die Gruppe A1 stehen, wobei $X^1$ $C_1$-$C_4$-Alkyl bedeutet, oder

bb) W für Sauerstoff, $R^4$ für Wasserstoff und A für die Gruppe A2 stehen, wobei $Y^2$ Cyclopropyl bedeutet, oder

cc) W für Sauerstoff, $R^4$ für Wasserstoff und A für die Gruppe A3 stehen, wobei $Z^3$ Halogen, Methyl oder Aethyl bedeutet, oder

dd) W für Sauerstoff, $R^4$ für Wasserstoff und A für die Gruppe $A^3$ stehen, wobei $Z^3$ und $Y^3$ zusammen eine Propylenbrücke bedeuten, oder

ee) W für Sauerstoff, $R^4$ für Wasserstoff und A für die Gruppe A5 stehen, wobei $X^5$ $C_1$-$C_4$-Alkoxy und $Z^5$ Methyl oder Aethyl bedeuten, oder

ff) W Sauerstoff, $R^4$ Wasserstoff und A für die Gruppe A4 stehen, wobei $X^4$ $C_1$-$C_4$-Alkyl oder $C_1$-$C_4$-Alkoxy bedeutet.

Als bevorzugte Einzelverbindungen der Formel I sind zu nennen :

N-(3,4-Dihydro-3-methyl-2,2-dioxo-1,2-benzoxathiin-8-ylsulfonyl)-N'-(4,6-dimethyl-1-oxo-1,3,5-triazin-2-yl)-harnstoff,

N-(3,4-Dihydro-3-methyl-2,2-dioxo-1,2-benzoxathiin-8-ylsulfonyl)-N'-(2,6-dimethoxy-5-methyl-pyrimidin-4-yl)-harnstoff und

N-(3,4-Dihydro-3-methyl-2,2-dioxo-1,2-benzoxathiin-8-ylsulfonyl)-N'-(5,6-dimethyl-1,2,4-triazin-3-yl)-harnstoff.

Die Herstellung der Verbindungen der Formel I erfolgt im allgemeinen nach den folgenden Methoden.

Nach einem ersten Verfahren werden die Verbindungen der Formel I erhalten, indem man ein Sulfonamid der Formel II

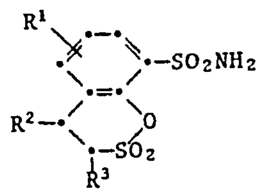

(II)

worin $R^1$, $R^2$ und $R^3$ die unter Formel I gegebene Bedeutung haben, in Gegenwart einer Base mit einem Carbamat der Formel III

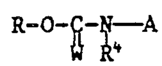

(III)

worin $R^4$, A und W die unter Formel I gegebene Bedeutung haben und R für Phenyl, Alkyl oder substituiertes Phenyl steht, umsetzt.

Nach einem zweiten Verfahren gelangt man zu Verbindungen der Formel I, indem man ein Sulfonylcarbamat der Formel IV

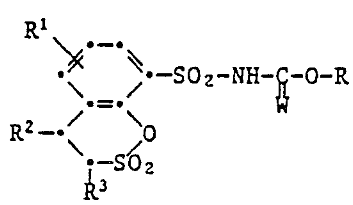

(IV)

worin $R^1$, $R^2$, $R^3$ und W die unter Formel I gegebene Bedeutung haben und R für Phenyl, Alkyl oder substituiertes Phenyl steht, mit einem Amin der Formel V

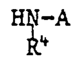

(V)

worin A und $R^4$ die unter Formel I gegebene Bedeutung haben, umsetzt.

Schliesslich kann man die Verbindungen der Formel I auch erhalten, indem man ein Sulfonylisocyanat der Formel VI

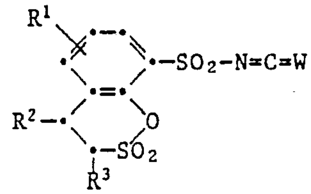

(VI)

worin $R^1$, $R^2$, $R^3$ und W die unter Formel I gegebenen Bedeutungen haben, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, Tetrachlorkohlenstoff, oder Chorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen —20° und + 120 °C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, 1,4-Diazabicyclo[2.2.2]-octan, 1,5-Diazabicyclo-[4.3.0]non-5-en oder 1,8-Diazabicyclo[5.4.0]undec-7-en geeignet. Als Basen können aber auch anorganische Basen wie Hydride wie Natrium-oder Calcicumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen gereinigt werden.

Die Zwischenprodukte der Formeln II, IV und VI und ihre Herstellung sind aus der EP-A-99339 bekannt.

Die als Ausgangsmaterialien verwendeten Aminopyrimidine, Aminotriazole und Aminotriazine der Formel V sowie entsprechende Carbamate der Formel III sind entweder bekannt oder sie lassen sich nach bekannten und in der Literatur beschriebenen Methoden erhalten.

Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Reis, Baumwolle, Soja und Mais befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d. h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z. B. die in der Landwirtschaft in tropischen Gegenden häufig als « cover crops » (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die « cover crops » jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei grösseren Aufwandmengen an Wirkstoffen der Formel I werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben, insbesondere Verfahren zur Unterdrueckung des Pflanzenwachstums ueber das 2-Blattstadium hinaus, wobei die Wirkstoffe preemergent angewandt werden.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensid-gemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazol-derivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1

bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxi-d-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgenden Publikationen beschrieben :

« Mc Cutcheon's Detergents and Emulsifiers Annual »
MC Publishing Corp., Ridgewood, New Jersey, 1981 ;
H. Stache, « Tensid-Taschenbuch », 2. Aufl., C. Hanser Verlag, München, Wien, 1981 ;
M. and J. Ash. « Encyclopedia of Surfactants », Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : (% = Gewichtsprozent).

| | |
|---|---|
| Emulgierbare Konzentrate : | |
| Aktiver Wirkstoff : | 1 bis 20 %, bevorzugt 5 bis 10 % |
| oberflächenaktive Mittel : | 5 bis 30 %, vorzugsweise 10 bis 20 % |
| flüssiges Trägermittel : | 50 bis 94 %, vorzugsweise 70 bis 85 % |
| Stäube : | |
| Aktiver Wirkstoff : | 0,1 bis 10 %, vorzugsweise 0,1 bis 1 % |
| festes Trägermittel : | 99,9 bis 90 %, vorzugsweise 99,9 bis 99 % |
| Suspensions-Konzentrate : | |
| Aktiver Wirkstoff : | 5 bis 75 %, vorzugsweise 10 bis 50 % |
| Wasser : | 94 bis 24 %, vorzugsweise 88 bis 30 % |
| oberflächenaktives Mittel : | 1 bis 40 %, vorzugsweise 2 bis 30 % |
| Benetzbares Pulver : | |
| Aktiver Wirkstoff : | 0,5 bis 90 %, vorzugsweise 1 bis 80 % |
| oberflächenaktives Mittel : | 0,5 bis 20 %, vorzugsweise 1 bis 15 % |
| festes Trägermittel : | 5 bis 95 %, vorzugsweise 15 bis 90 % |
| Granulate : | |
| Aktiver Wirkstoff : | 0,5 bis 30 %, vorzugsweise 3 bis 15 % |
| festes Trägermittel : | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### Beispiel HI

N-(3,4-Dihydro-3-methyl-2,2-dioxo-1,2-benzoxathiin-8-ylsulfonyl)-N'-(4-cyclopropyl-6-methyl-pyrimidin-2-yl)-harnstoff. (Verbindung 2.01)

a) N-(3,4-Dihydro-3-methyl-2,2-dioxo-1,2-benzoxathiin-8-ylsulfonyl)-N'-methylharnstoff.

Eine Lösung von 24,96 g 3,4-Dihydro-3-methyl-2,2-dioxo-1,2-benzoxathiin-8-yl-sulfonamid in 120 ml absolutem Acetonitril wird auf + 8 °C gekühlt. Nach Zusatz von 6 ml Methylisocyanat lässt man 13,4 ml 1,8-Diazabicyclo[5.4.0]undec-7-en in 15 ml Acetonitril bei 10 °C innerhalb von 15 Minuten zutropfen. Die

Reaktionsmischung wird für eine weitere Stunde bei Raumtemperatur gerührt. Anschliessend wird das Gemisch mit 180 ml Wasser verdünnt. Durch tropfenweise Zugabe von 48 ml 2 N Salzsäure wird das Produkt ausgefällt. Durch Abtrennen und Trocknen des Niederschlages erhält man 26,4 g N-(3,4-Dihydro-3-methyl-2,2-dioxo-1,2-benzoxathiin-8-ylsulfonyl)-N'-methyl-harnstoff, Smp. 235-238 °C.

b) 3,4-Dihydro-3-methyl-2,2-dioxo-1,2-benzoxathiin-8-sulfonylisocyanat.

14,3 g N-(3,4-Dihydro-3-methyl-2,2-dioxo-1,2-benzoxathiin-8-ylsulfonyl)-N'-methyl-harnstoff werden in 143 ml trockenem Dichlorbenzol suspendiert. Bei einer Tempertur von 140 °C leitet man in die Mischung innerhalb von 75 Minuten 9,5 g Phosgen ein. Anschliessend wird durch das Reaktionsgemisch für 20 Minuten trockener Stickstoff durchgeblasen. Die Lösung wird unter Feuchtigkeitsausschluss bei 90 °C/20 mbar eingedampft. Man erhält 15,1 g 3,4-Dihydro-3-methyl-2,2-dioxo-1,2-benzoxathiin-8-ylsulfonyl-isocyanat in Form eines Oeles, das in warmem absolutem Dioxan gelöst und ohne weitere Lagerung in der Folgereaktion umgesetzt wird.

c) Zu einer Mischung von 3,72 g 2-Amino-4-cyclopropyl-6-methyl-pyrimidin in 100 ml absolutem Dioxan lässt man 66,5 ml einer 10,78 %igen Lösung von 3,4-Dihydro-3-methyl-2,2-dioxo-1,2-benzoxathiin-8-ylsulfonylisocyanat in Dioxan innerhalb von 5 Minuten bei Raumtemperatur zutropfen. Die Mischung wird für weitere 18 Stunden gerührt und dann eingedampft. Der Rückstand wird durch Verreiben mit einem Aethylacetat/Benzin-Gemisch (1 : 2) kristallisiert. Man erhält 11,2 g N-(3,4-Dihydro-3-methyl-2,2-dioxo-1,2-benzoxathiin-8-ylsulfonyl)-N'-(4-cyclopropyl-6-methyl-pyrimidin-2-yl)harnstoff, Smp. 216-217 °C.

In analoger Weise werden die in den angeschlossenen Tabellen aufgelisteten Verbindungen der Formel I hergestellt.

Tabelle 1

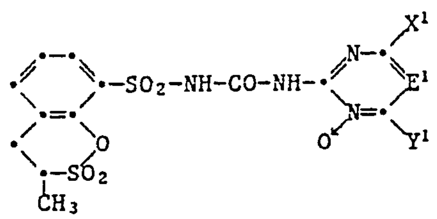

| Verb. Nr. | $E^1$ | $X^1$ | $Y^1$ | Smp. [°C] |
|---|---|---|---|---|
| 1.01 | CH | $CH_3$ | $OCH_3$ | 148-150 |
| 1.02 | CH | $CH_3$ | $CH_3$ | 181-182 |
| 1.03 | N | $CH_3$ | $CH_3$ | 162-163 |
| 1.04 | CH | $CH_3$ | $OC_2H_5$ | |
| 1.05 | CH | $OCH_3$ | $OCH_3$ | |
| 1.06 | CH | $N(CH_3)_2$ | $OCH_3$ | |

Tabelle 2

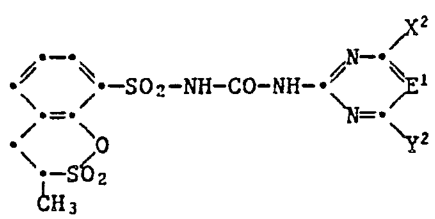

| Verb. Nr. | $E^1$ | $X^2$ | $Y^2$ | Smp. [°C] |
|---|---|---|---|---|
| 2.01 | CH | $CH_3$ | Cyclopropyl | 216-217 |
| 2.02 | N | $OCH_3$ | Cyclopropyl | 186-187 |
| 2.03 | CH | $OCH_3$ | Cyclopropyl | |
| 2.04 | N | $CH_3$ | Cyclopropyl | |

Tabelle 3

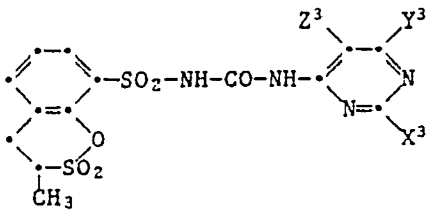

| Verb. Nr. | $X^3$ | $Y^3$ | $Z^3$ | Smp. [°C] |
|---|---|---|---|---|
| 3.01 | $OCH_3$ | $OCH_3$ | Cl | 227-228 |
| 3.02 | $CH_3$ | $OCH_3$ | Cl | 221-223 |
| 3.03 | $OCH_3$ | $CH_3$ | Br | 212-213 |
| 3.04 | $OCH_3$ | $-CH_2-CH_2-CH_2-$ | | 221-222 |
| 3.05 | $OCH_3$ | $OCH_3$ | $CH_3$ | 215-216 |
| 3.06 | $OCH_3$ | $OCH_3$ | F | |

Tabelle 4

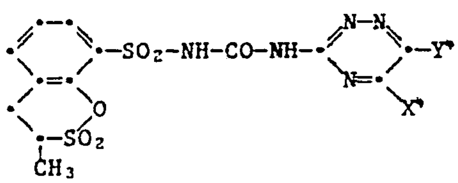

| Verb. Nr. | $X^4$ | $Y^4$ | Smp. [°C] |
|---|---|---|---|
| 4.01 | $CH_3$ | $CH_3$ | 161-162 |
| 4.02 | $CH_3$ | H | |
| 4.03 | $OCH_3$ | H | |
| 4.04 | $OCH_3$ | $OCH_3$ | |

Tabelle 5

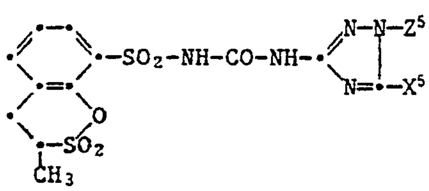

| Verb. Nr. | $X^5$ | $Z^5$ | Smp. [°C] |
|---|---|---|---|
| 5.01 | $OCH_3$ | $CH_3$ | 198 |
| 5.02 | $CH_3$ | $CH_3$ | |

Tabelle 6

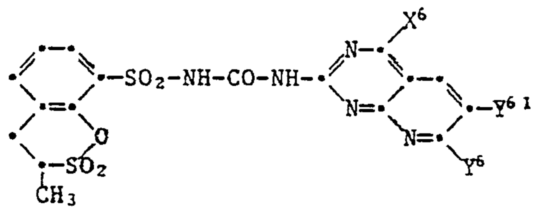

| Verb. Nr. | $X^6$ | $Y^6$ | $Y^{6\,1}$ |
|---|---|---|---|
| 6.01 | $OCH_3$ | $CH_3$ | H |
| 6.02 | $OCH_3$ | H | $CH_3$ |
| 6.03 | $CH_3$ | $CH_3$ | H |

Tabelle 7

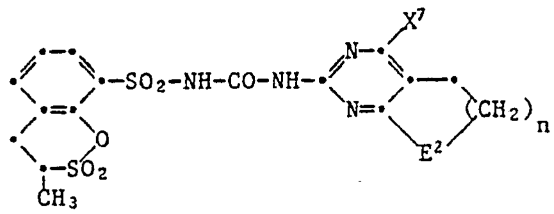

| Verb. Nr. | $E^2$ | n | $X^7$ |
|---|---|---|---|
| 7.01 | O | 1 | $CH_3$ |
| 7.02 | O | 1 | $OCH_3$ |
| 7.03 | $CH_2$ | 1 | $OCH_3$ |
| 7.04 | O | 2 | $OCH_3$ |
| 7.05 | $CH_2$ | 2 | $OCH_3$ |
| 7.06 | $CH_2$ | 1 | $CH_3$ |

Tabelle 8

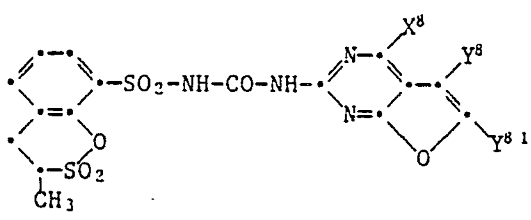

| Verb. Nr. | $X^8$ | $Y^8$ | $Y^{8\,1}$ |
|---|---|---|---|
| 8.01 | $CH_3$ | H | $CH_3$ |
| 8.02 | $OCH_3$ | H | $CH_3$ |
| 8.03 | $OCH_3$ | H | H |
| 8.04 | $CH_3$ | H | $CH_3$ |
| 8.05 | $OCH_3$ | $CH_3$ | $CH_3$ |

Formulierungsbeispiele

Beispiel F 1

Formulierungsbeispiele für Wirkstoffe der Formel I (% = Gewichtsprozent)

| a) Spritzpulver | a) | b) | c) |
|---|---|---|---|
| Wirkstoff | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther /7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses

Gemisch wird extrudiert und anschliessend im Luftstrom getrocknet.

| | |
|---|---|
| e) Umhüllungs-Granulat | |
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält an staubfreie Umhüllungs-Granulate.

| f) Suspensions-Konzentrat | a) | b) |
|---|---|---|
| Wirkstoff | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| | |
|---|---|
| g) Salzlösung | |
| Wirkstoff | 5 % |
| Isopropylamin | 1 % |
| Octylphenolpolyäthylenglykoläther (78 Mol AeO) | 3 % |
| Wasser | 91 % |

## Biologische Beispiele

### Beispiel B 1

### Herbizidwirkung vor dem Auflaufen der Pflanzen

Kunststofftöpfe werden mit expandiertem Vermiculit (Dichte: 0,135 g/cm³, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20 °C, einer Beleuchtung von ca. 20 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5.Tag wird dem Giesswasser 0,5 % eines handelsüblichen Flüssigdüngers (® Greenzit, ex Ciba-Geigy) zugesetzt. 12 Tage nach der Aussaat wird der Versuch ausgewertet und die Wirkung auf die Versuchspflanzen nach dem folgenden Massstab bewertet:

1 : Pflanze nicht gekeimt oder total abgestorben
2-3 : sehr starke Wirkung
4-6 : mittlere Wirkung
7-8 : schwache Wirkung
9 : keine Wirkung (wie unbehandelte Kontrolle).

pre-emergente Wirkung:
Konzentration der Wirkstoffemulsion: 70,8 ppm

| Testpflanze / Wirkstoff Nr | Nasturtium | Stellaria | Agrostis | Digitaria |
|---|---|---|---|---|
| 1.01 | 3 | 4 | 4 | 3 |
| 1.02 | 4 | 3 | 4 | 3 |
| 1.03 | 1 | 1 | 1 | 1 |
| 2.01 | 4 | 4 | 4 | 4 |
| 2.02 | 4 | 4 | 4 | 4 |
| 3.01 | 4 | 4 | 4 | 4 |
| 3.02 | 4 | 3 | 3 | 3 |
| 3.03 | 4 | 4 | 4 | 4 |
| 3.04 | 4 | 3 | 4 | 3 |
| 3.05 | 2 | 1 | 2 | 1 |
| 4.01 | 1 | 1 | 1 | 1 |
| 5.01 | 3 | 3 | 3 | 3 |

Beispiel B 2

Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 600 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt werden.

Beispiel B 3

Wuchsregulierung an Sojabohnen

In Kunststoffbehältern mit einem Erde-Torf-Sandgemisch im Verhältnis 6 : 3 : 1 werden Sojabohnen der Sorte « Hark » angesät und in eine Klimakammer gegeben. Durch optimale Temperaturwahl, Beleuchtung, Dünger zugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel B 4

Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel B 5

Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

## Patentansprüche

1. N-(3,4-Dihydro-2,2-dioxo-1,2-benzoxathiin-8-ylsulfonyl)-N'-pyrimidinyl-, N'-triazolyl- oder N'-triazinyl-harnstoffe der Formel I

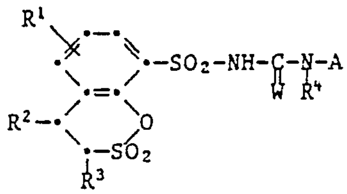

worin

$R^1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_5$-Alkoxyalkoxy,

$R^2$ und $R^3$ unabhängig voneinander Wasserstoff oder $C_1$-$C_4$-Alkyl,

$R^4$ Wasserstoff, Methyl oder Aethyl,

W Sauerstoff oder Schwefel, und

A einen Rest

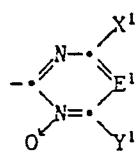

A1

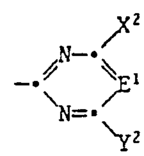

A2

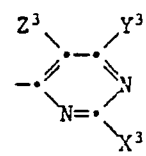

A3

,

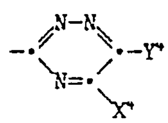

A4

,

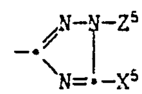

A5

,

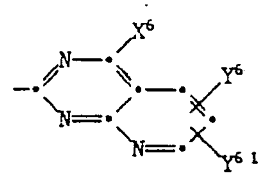

A6

,

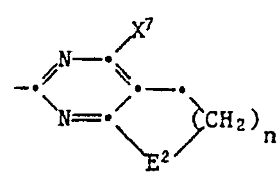

A7

oder

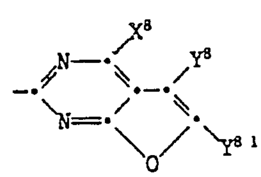

A8

bedeuten, wobei

$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halogenalkyl, $C_1$-$C_4$-Alkoxy, $C_1$-$C_4$-Alkyl, Dimethylamino, Methylamino, Aethylamino, Amino oder $C_2$-$C_4$-Alkoxyalkyl,

$E^1$ für Stickstoff oder die Methinbrücke,

$E^2$ für Sauerstoff oder die Methylenbrücke,

$Y^2$ für Cyclopropyl, Dimethoxymethyl, Diäthoxymethyl

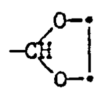

oder

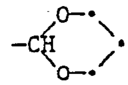

$Y^3$ für Methyl, Methoxy, Aethyl, Aethoxy, $C_1$-$C_2$-Halogenalkyl oder $C_1$-$C_2$-Halogenalkoxy,

$Y^4$ für Wasserstoff, Methoxy, Aethoxy, Halogen oder $C_1$-$C_4$-Alkyl,

$Y^6$ für $Y^{6\ 1}$ unabhängig voneinander für Wasserstoff, $C_1$-$C_4$-Alkyl, Halogen, Methoxy oder Methylthio,

$Y^8$ und $Y^{8\ 1}$ unabhängig voneinander für Wasserstoff oder Methyl,

n für eins oder zwei,

$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,

$Z^3$ und $Y^3$ zusammen für eine $C_2$-$C_4$-Alkylenbrücke oder eine durch Sauerstoff unterbrochene $C_2$-$C_4$-Alkylenbrücke, und

$Z^5$ für Methyl oder Aethyl stehen,

sowie den Salzen dieser Verbindungen; mit der Massgabe, dass $R^3$ $C_1$-$C_4$-Alkyl bedeutet wenn A für einen der Reste A2, A5, A7 oder A8 steht.

2. Verbindungen gemäss Anspruch 1, dadurch gekennzeichnet, dass $R^1$ und $R^2$ Wasserstoff und $R^3$ Methyl bedeuten.

3. Verbindungen gemäss Anspruch 1, dadurch gekennzeichnet, dass W Sauerstoff bedeutet.

4. Verbindungen gemäss Anspruch 1, dadurch gekennzeichnet, dass $R^4$ Wasserstoff ist.

5. Verbindungen gemäss Anspruch 1, dadurch gekennzeichnet, dass W Sauerstoff und $R^4$ Wasserstoff bedeuten.

6. Verbindungen gemäss Anspruch 1, dadurch gekennzeichnet, dass W für Sauerstoff, $R^4$ für Wasserstoff und A für die Gruppe A1 stehen, wobei $X^1$ $C_1$-$C_4$-Alkyl bedeutet.

7. Verbindungen gemäss Anspruch 1, dadurch gekennzeichnet, dass W für Sauerstoff, $R^4$ für Wasserstoff und A für die Gruppe A2 stehen, wobei $Y^2$ Cyclopropyl bedeutet.

8. Verbindungen gemäss Anspruch 1, dadurch gekennzeichnet, dass W für Sauerstoff, $R^4$ für Wasserstoff und A für die Gruppe A3 stehen, wobei $Z^3$ Halogen, Methyl oder Aethyl bedeutet.

9. Verbindungen gemäss Anspruch 1, dadurch gekennzeichnet, dass W für Sauerstoff, $R^4$ für Wasserstoff und A für die Gruppe A3 stehen, wobei $Z^3$ und $Y^3$ zusammen eine Propylenbrücke bedeuten.

10. Verbindungen gemäss Anspruch 1, dadurch gekennzeichnet, dass W für Sauerstoff, $R^4$ für Wasserstoff und A für die Gruppe A5 stehen, wobei $X^5$ $C_1$-$C_4$-Alkoxy und $Z^5$ Methyl oder Aethyl bedeuten.

11. Verbindungen gemäss Anspruch 1, dadurch gekennzeichnet, dass W Sauerstoff, $R^4$ Wasserstoff und A für die Gruppe A4 stehen, wobei $X^4$ $C_1$-$C_4$-Alkyl oder $C_1$-$C_4$-Alkoxy bedeutet.

12. N-(3,4-Dihydro-3-methyl-2,2-dioxo-1,2-benzoxathiin-8-ylsulfonyl)-N'-(4,6-dimethyl-1-oxo-1,3,5-triazin-2-yl)-harnstoff gemäss Anspruch 1.

13. N-(3,4-Dihydro-3-methyl-2,2-dioxo-1,2-benzoxathiin-8-ylsulfonyl)-N'-(2,6-dimethoxy-5-methyl-pyrimidin-4-yl)-harnstoff gemäss Anspruch 1.

14. N-(3,4-Dihydro-3-methyl-2,2-dioxo-1,2-benzoxathiin-8-ylsulfonyl)-N'-(5,6-dimethyl-1,2,4-triazin-3-yl)-harnstoff gemäss Anspruch 1.

15. Verfahren zur Herstellung der Verbindungen der Formel I nach Anspruch 1, dadurch gekennzeichnet, dass man ein Sulfonamid der Formel II

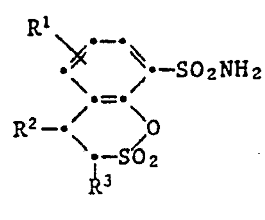

(II)

worin $R^1$, $R^2$ und $R^3$ die unter Formel I gegebene Bedeutung haben, in Gegenwart einer Base mit einem Carbamat der Formel III

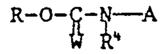

(III)

worin A, $R^4$ und W die unter Formel I gegebene Bedeutung haben und R für Phenyl, Alkyl oder substituiertes Phenyl steht, umsetzt und gegebenenfalls in ein Salz überführt.

16. Verfahren zur Herstellung der Verbindungen der Formel I nach Anspruch 1, dadurch gekennzeichnet, dass man ein Sulfonylcarbamat der Formel IV

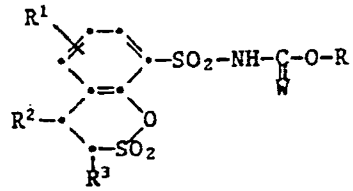

(IV)

worin $R^1$, $R^2$, $R^3$ und W die unter Formel I gegebene Bedeutung haben und R für Phenyl, Alkyl oder substituiertes Phenyl steht, mit einem Amin der Formel V

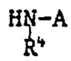

(V)

worin A und $R^4$ die unter Formel I gegebene Bedeutung haben, umsetzt und gegebenenfalls in ein Salz überführt.

17. Verfahren zur Herstellung der Verbindungen der Formel I, nach Anspruch 1, dadurch gekennzeichnet, dass man ein Sulfonylisocyanat der Formel VI

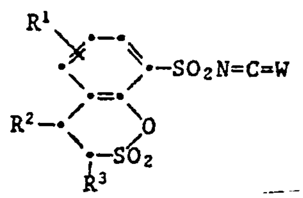

(VI)

worin $R^1$, $R^2$, $R^3$ und W die unter Formel I gegebene Bedeutung haben, mit einem Amin der Formel V

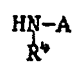

(V)

worin A und $R^4$ die unter Formel I gegebene Bedeutung haben, umsetzt und gegebenenfalls in ein Salz überführt.

18. Verfahren zur Herstellung von Additionssalzen der Formel I gemäss einem der Ansprüche 15 bis 17, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

19. Ein herbizides und den Pflanzenwuchs hemmendes Mittel, dadurch gekennzeichnet, dass es neben Träger- und/oder anderen Zuschlagstoffen als Wirkstoff mindestens einen substituierten Sulfonylharnstoff der Formel I, Anspruch 1, enthält.

20. Verfahren zur Bekämpfung unerwünschten Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

21. Verfahren zur Hemmung des Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

22. Verfahren zur Beeinflussung des Pflanzenwachstums zum Zweck der Ertragssteigerung, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

23. Verfahren gemäss Anspruch 20, zur selektiven pre- oder post-emergenten Bekämpfung von Unkräutern in Nutzpflanzenkulturen.

24. Verfahren gemäss Anspruch 21, zur Unterdrückung des Pflanzenwachstums über das 2-Blattstadium hinaus, dadurch gekennzeichnet, dass die Wirkstoffe preemergent angewendet werden.

25. Verbindungen gemäss Anspruch 1, dadurch gekennzeichnet, dass

$R^1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxyalkoxy,

$R^2$ und $R^3$ unabhängig voneinander Wasserstoff oder $C_1$-$C_4$-Alkyl,

$R^4$ Wasserstoff, Methyl oder Aethyl,

W Sauerstoff oder Schwefel, und

A einen Rest

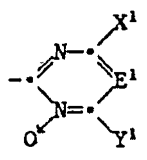

A1

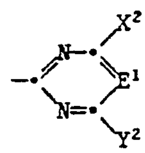

A2

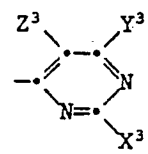

A3

,

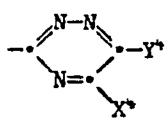

A4

oder

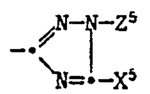

A5

,

bedeuten, wobei

$X^1$, $X^2$, $X^3$, $X^4$, $X^5$ und $Y^1$ unabhängig voneinander für Halogen, $C_1$-$C_4$-Halogenalkoxy, $C_1$-$C_4$-Halogenalkyl, $C_1$-$C_4$-Alkoxy, $C_1$-$C_4$-Alkyl, Dimethylamino, Methylamino, Aethylamino, Amino oder $C_2$-$C_4$-Alkoxyalkyl,

$E^1$ für Stickstoff oder die Methinbrücke,

$Y^2$ für Cyclopropyl, Dimethoxymethyl, Diäthoxymethyl

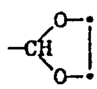

oder

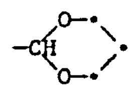

,

$Y^3$ für Methyl, Methoxy, Aethyl, Aethoxy, $C_1$-$C_2$-Halogenalkyl oder $C_1$-$C_2$-Halogenalkoxy,

$Y^4$ für Wasserstoff, Methoxy, Aethoxy, Halogen oder $C_1$-$C_4$-Alkyl,

$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,

$Z^3$ und $Y^3$ zusammen für eine $C_2$-$C_4$-Alkylenbrücke oder eine durch Sauerstoff unterbrochene $C_2$-$C_4$-Alkylenbrücke, und

$Z^5$ für Methyl oder Aethyl stehen,

sowie den Salzen dieser Verbindungen; mit der Massgabe, dass $R^3$ $C_1$-$C_4$-Alkyl bedeutet wenn A für einen der Reste A2 oder A5 steht.

## Claims

1. N-(3,4-dihydro-2,2-dioxo-1,2-benzoxathiin-8-ylsulfonyl)N'-pyrimidinyl-, N'-triazolyl- or N'-triazinylureas of formula I

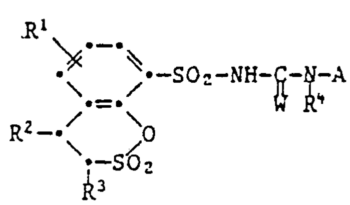

(I)

wherein

$R^1$ is hydrogen, halogen, nitro, $C_1$-$C_4$alkyl, $C_1$-$C_4$haloalkyl, $C_1$-$C_4$alkoxy, $C_1$-$C_4$haloalkoxy, $C_1$-$C_4$alkoxycarbonyl, $C_1$-$C_4$alkylthio, $C_1$-$C_4$alkylsulfinyl, $C_1$-$C_4$alkylsulfonyl or $C_2$-$C_5$alkoxyalkoxy,
$R^2$ and $R^3$ are each independently of the other hydrogen or $C_1$-$C_4$alkyl,
$R^4$ is hydrogen, methyl or ethyl,
W is oxygen or sulfur, and
A is a radical

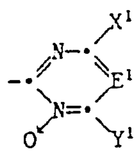
A1

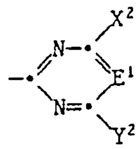
A2

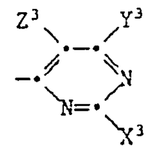
A3
,

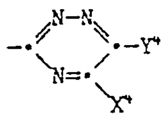
A4
,

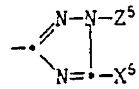
A5
,

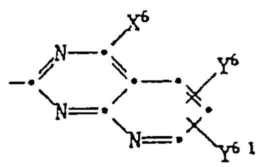
A6
,

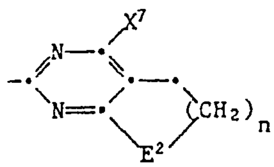
A7
or

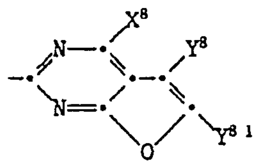
A8

in which formulae
$X^1$, $X^2$, $X^3$, $X^4$, $X^5$, $X^6$, $X^7$, $X^8$ and $Y^1$ are each independently halogen, $C_1$-$C_4$haloalkoxy, $C_1$-$C_4$haloalkyl, $C_1$-$C_4$alkoxy, $C_1$-$C_4$alkyl, dimethylamino, methylamino, ethylamino, amino or $C_2$-$C_4$alkoxyalkyl,
$E^1$ is nitrogen or the methine bridge,
$E^2$ is oxygen or the methylene bridge,
$Y^2$ is cyclopropyl, dimethoxymethyl, diethoxymethyl,

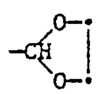
or
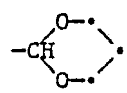
,

$Y^3$ is methyl, methoxy, ethyl, ethoxy, $C_1$-$C_2$haloalkyl or $C_1$-$C_2$haloalkoxy,
$Y^4$ is hydrogen, methoxy, ethoxy, halogen or $C_1$-$C_4$alkyl,
$Y^6$ and $Y^{6\,1}$ are each independently or the other hydrogen, $C_1$-$C_4$alkyl, halogen, methoxy or methylthio,
$Y^8$ and $Y^{8\,1}$ are each independently of the other hydrogen or methyl,
n is 1 or 2,
$Z^3$ is hydrogen, methyl, ethyl, methoxy, ethoxy, methoxycarbonyl, ethoxycarbonyl, halogen, cyano, nitro, methylthio, methylsulfinyl or methylsulfonyl,
$Z^3$ and $Y^3$ together are a $C_2$-$C_4$alkylene bridge or a $C_2$-$C_4$alkylene bridge which is interrupted by oxygen, and
$Z^5$ is methyl or ethyl,
and the salts of these compounds ; with the proviso that $R^3$ is $C_1$-$C_4$alkyl if A is one of the radicals A2, A5, A7 or A8.

2. Compounds according to claim 1, wherein $R^1$ and $R^2$ are hydrogen and $R^3$ is methyl.

3. Compounds according to claim 1, wherein W is oxygen.

4. Compounds according to claim 1, wherein $R^4$ is hydrogen.

5. Compounds according to claim 1, wherein W is oxygen and $R^4$ is hydrogen.

6. Compounds according to claim 1, wherein W is oxygen, $R^4$ is hydrogen and A is the group A1 in which $X^1$ is $C_1$-$C_4$alkyl.

7. Compounds according to claim 1, wherein W is oxygen, $R^4$ is hydrogen and A is the group A2 in which $Y^2$ is cyclopropyl.

8. Compounds according to claim 1, wherein W is oxygen, $R^4$ is hydrogen and A is the group A3 in which $Z^3$ is halogen, methyl or ethyl.

9. Compounds according to claim 1, wherein W is oxygen, $R^4$ is hydrogen and A is the group A3 in which $Z^3$ and $Y^3$ together are a propylene bridge.

10. Compounds according to claim 1, wherein W is oxygen, $R^4$ is hydrogen and A is the group A5 in which $X^5$ is $C_1$-$C_4$alkoxy and $Z^5$ is methyl or ethyl.

11. Compounds according to claim 1, wherein W is oxygen, $R^4$ is hydrogen and A is the group A4 in which $X^4$ is $C_1$-$C_4$alkyl or $C_1$-$C_4$alkoxy.

12. N-(3,4-dihydro-3-methyl-2,2-dioxo-1,2-benzoxathiin-8-ylsulfonyl)-N'-(4,6-dimethyl-1-oxo-1,3,5-triazin-2-yl)-urea according to claim 1.

13. N-(3,4-dihydro-3-methyl-2,2-dioxo-1,2-benzoxathiin-8-ylsulfonyl)-N'-(2,6-dimethoxy-5-methyl-pyrimidin-4-yl)-urea according to claim 1.

14. N-(3,4-dihydro-3-methyl-2,2-dioxo-1,2-benzoxathiin-8-ylsulfonyl)-N'-(5,6-dimethyl-1,2,4-triazin-3-yl)-urea according to claim 1.

15. A process for the preparation of compounds of formula I according to claim 1, which process comprises reacting a sulfonamide of formula II

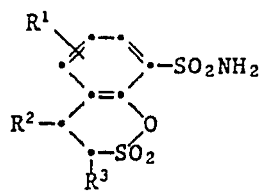 (II)

wherein $R^1$, $R^2$ and $R^3$ are as defined for formula I, with a carbamate of formula III

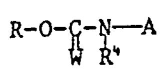 (III)

wherein A, $R^4$ and W are as defined for formula I and R is phenyl, alkyl or substituted phenyl, in the presence of a base, and, if desired, converting the resultant compound of formula I into a salt.

16. A process for the preparation of compounds of formula I according to claim 1, which process comprises reacting a sulfonylcarbamate of formula IV

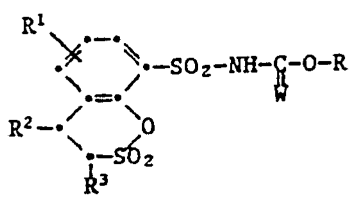 (IV)

wherein $R^1$, $R^2$, $R^3$ and W are as defined for formula I and R is phenyl, alkyl or substituted phenyl, with an amine of formula V

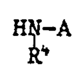 (V)

wherein A and $R^4$ are as defined for formula I, and, if desired, converting a resultant compound of formula I into a salt.

17. A process for the preparation of compounds of formula I according to claim 1, which process comprises reacting a sulfonylisocyanate of formula VI

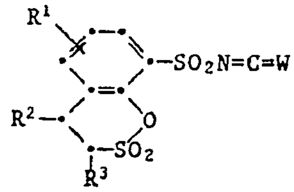

(VI)

wherein $R^1$, $R^2$, $R^3$ and W are as defined for formula I, with an amine of formula V

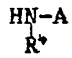

(V)

wherein A and $R^4$ are as defined for formula I, and, if desired, converting the resultant compound of formula I into a salt.

18. A process for the preparation of addition salts of formula I according to any one of claims 15 to 17, which process comprises reacting a sulfonylurea of formula I with an amine, an alkali metal hydroxide or an alkaline earth metal hydroxide or with a quaternary ammonium base.

19. A herbicidal and plant growth inhibiting composition which contains, as active ingredient, at least one substituted sulfonylurea of formula I, claim 1, together with carriers and/or other adjuvants.

20. A method of controlling undesired plant growth, which method comprises applying to the plants or to the locus thereof an effective amount of an active ingredient of formula I, according to claim 1, or of a composition containing that active ingredient.

21. A method of inhibiting plant growth, which method comprises applying to the plants or to the locus thereof an effective amount of an active ingredient of formula I, according to claim 1, or of a composition containing that active ingredient.

22. A method of influencing plant growth for increasing yield, which method comprises applying to the plants or to the locus thereof an effective amount of an active ingredient of formula I, according to claim 1, or of a composition containing that active ingredient.

23. A method according to claim 20 of selectively controlling weeds pre- or post-emergence in crops of useful plants.

24. A method according to claim 21 of suppressing plant growth beyond the 2-leaf stage, which method comprises applying the active ingredients pre-emergence.

25. Compounds according to claim 1, wherein

$R^1$ is hydrogen, halogen, nitro, $C_1$-$C_4$alkyl, $C_1$-$C_4$haloalkyl, $C_1$-$C_4$alkoxy, $C_1$-$C_4$haloalkoxy, $C_1$-$C_4$alkoxycarbonyl, $C_1$-$C_4$alkylthio, $C_1$-$C_4$alkylsulfinyl, $C_1$-$C_4$alkylsulfonyl or $C_2$-$C_5$alkoxyalkoxy,

$R^2$ and $R^3$ are each independently of the other hydrogen or $C_1$-$C_4$alkyl,

$R^4$ is hydrogen, methyl or ethyl,

W is oxygen or sulfur, and

A is a radical

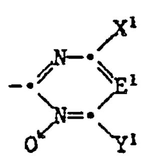

A1

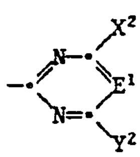

A2

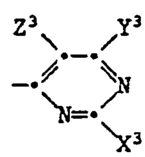

A3

,

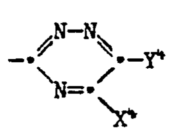

A4

or

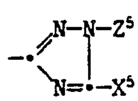

A5

,

in which formulae

$X^1$, $X^2$, $X^3$, $X^4$, $X^5$ and $Y^1$ are each independently halogen, $C_1$-$C_4$haloalkoxy, $C_1$-$C_4$haloalkyl, $C_1$-$C_4$alkoxy, $C_1$-$C_4$alkyl, dimethylamino, methylamino, ethylamino, amino or $C_2$-$C_4$alkoxyalkyl,

$E^1$ is nitrogen or the methine bridge,
$Y^2$ is cyclopropyl, dimethoxymethyl, diethoxymethyl,

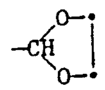 or 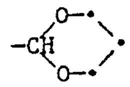 ,

$Y^3$ is methyl, methoxy, ethyl, ethoxy, $C_1$-$C_2$haloalkyl or $C_1$-$C_2$haloalkoxy,
$Y^4$ is hydrogen, methoxy, ethoxy, halogen or $C_1$-$C_4$alkyl,
$Z^3$ is hydrogen, methyl, ethyl, methoxy, ethoxy, methoxycarbonyl, ethoxycarbonyl, halogen, cyano, nitro, methylthio, methylsulfinyl or methylsulfonyl,
$Z^3$ and $Y^3$ together are a $C_2$-$C_4$alkylene bridge or a $C_2$-$C_4$alkylene bridge which is interrupted by oxygen, and
$Z^5$ is methyl or ethyl,
and the salts of these compounds ; with the proviso that $R^3$ is $C_1$-$C_4$alkyl if A is one of the radicals A2 or A5.

## Revendications

1. N-(3,4-dihydro-2,2-dioxo-1,2-benzoxathiine-8-ylsulfonyl)-N'-pyrimidinyl-, N'-triazolyl- ou N'-triazinyl-urées de formule I

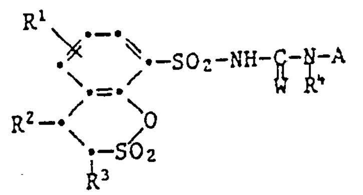 (I)

dans laquelle
$R^1$ représente l'hydrogène, un halogène, un groupe nitro, alkyle en $C_1$-$C_4$, halogénoalkyle en $C_1$-$C_4$, alcoxy en $C_1$-$C_4$, halogénoalcoxy en $C_1$-$C_4$ (alcoxy en $C_1$-$C_4$)-carbonyle, alkylthio en $C_1$-$C_4$, alkylsulfinyle en $C_1$-$C_4$, alkylsulfonyle en $C_1$-$C_4$ ou alcoxyalcoxy en $C_2$-$C_5$,
$R^2$ et $R^3$ représentent chacun, indépendamment l'un de l'autre, l'hydrogène ou un groupe alkyle en $C_1$-$C_4$,
$R^4$ représente l'hydrogène, un groupe méthyle ou éthyle,
W représente l'oxygène ou le soufre, et
A représente un groupe

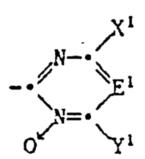
A1

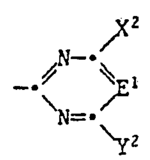
A2

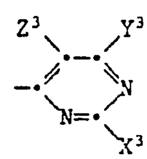 ,
A3

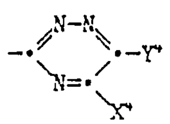 ,
A4

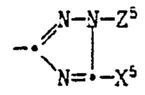 ,
A5

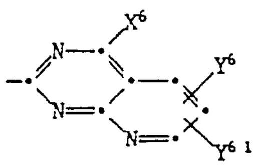 ,
A6

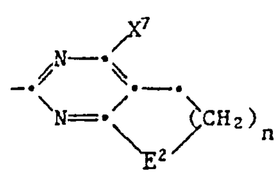 ou
A7

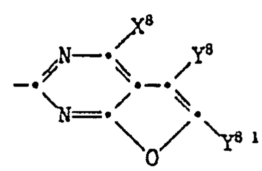
A8

dans lesquels

$X^1$, $X^2$, $X^3$, $X^4$, $X^5$, $X^6$, $X^7$, $X^8$ et $Y^1$ représentent chacun, indépendamment les uns des autres, un halogène, un groupe halogénoalcoxy en $C_1$-$C_4$, halogénoalkyle en $C_1$-$C_4$, alcoxy en $C_1$-$C_4$, alkyle en $C_1$-$C_4$, diméthylamino, méthylamino, éthylamino, amino ou alcoxyalkyle en $C_2$-$C_4$,

$E^1$ représente l'azote ou le pont méthine,

$E^2$ représente l'oxygène ou le pont méthylène,

$Y^2$ représente un groupe cyclopropyle, diméthoxyméthyle, diéthoxyméthyle

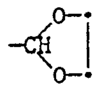 ou 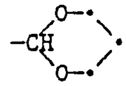 ,

$Y^3$ représente un groupe méthyle, méthoxy, éthyle, éthoxy, halogénoalkyle en $C_1$-$C_2$ ou halogénoalcoxy en $C_1$-$C_2$,

$Y^4$ représente l'hydrogène, un groupe méthoxy, éthoxy, un halogène ou un groupe alkyle en $C_1$-$C_4$,

$Y^6$ et $Y^{6\ 1}$ représentent chacun, indépendamment l'un de l'autre, l'hydrogène, un groupe alkyle en $C_1$-$C_4$, un halogène, un groupe méthoxy ou méthylthio,

$Y^8$ et $Y^{8\ 1}$ représentent chacun, indépendamment l'un de l'autre, l'hydrogène ou un groupe méthyle,

n est égal à 1 ou 2,

$Z^3$ représente l'hydrogène, un groupe méthyle, éthyle, méthoxy, éthoxy, méthoxycarbonyle, éthoxycarbonyle, un halogène, un groupe cyano, nitro, méthylthio, méthylsulfinyle ou méthylsulfonyle,

$Z^3$ et $Y^3$ forment ensemble un pont alkylène en $C_2$-$C_4$ ou un pont alkylène en $C_2$-$C_4$ interrompu par l'oxygène, et

$Z^5$ représente un groupe méthyle ou éthyle, ainsi que les sels de ces composés ; sous réserve que $R^3$ doit représenter un groupe alkyle en $C_1$-$C_4$ lorsque A représente l'un des groupes $A^2$, $A^5$, $A^7$ ou $A^8$.

2. Composés selon la revendication 1, caractérisés en ce que $R^1$ et $R^2$ représentent l'hydrogène et $R^3$ un groupe méthyle.

3. Composés selon la revendication 1, caractérisés en ce que W représente l'oxygène.

4. Composés selon la revendication 1, caractérisés en ce que $R^4$ représente l'hydrogène.

5. Composés selon la revendication 1, caractérisés en ce que W représente l'oxygène et $R^4$ l'hydrogène.

6. Composés selon la revendication 1, caractérisés en ce que W représente l'oxygène, $R^4$ l'hydrogène et A le groupe A1 dans lequel $X^1$ représente un groupe alkyle en $C_1$-$C_4$.

7. Composés selon la revendication 1, caractérisés en ce que W représente l'oxygène, $R^4$ l'hydrogène et A le groupe A2 dans lequel $Y^2$ représente un groupe cyclopropyle.

8. Composés selon la revendication 1, caractérisés en ce que W représente l'oxygène, $R^4$ l'hydrogène et A le groupe A3 dans lequel $Z^3$ représente un halogène, un groupe méthyle ou éthyle.

9. Composés selon la revendication 1, caractérisés en ce que W représente l'oxygène, $R^4$ l'hydrogène et A le groupe A3 dans lequel $Z^3$ et $Y^3$ forment ensemble un pont propylène.

10. Composés selon la revendication 1, caractérisés en ce que W représente l'oxygène, $R^4$ l'hydrogène et A le groupe A5 dans lequel $X^5$ représente un groupe alcoxy en $C_1$-$C_4$ et $Z^5$ un groupe méthyle ou éthyle.

11. Composés selon la revendication 1, caractérisés en ce que W représente l'oxygène, $R^4$ l'hydrogène et A le groupe A4 dans lequel $X^4$ représente un groupe alkyle en $C_1$-$C_4$ ou alcoxy en $C_1$-$C_4$.

12. La N-(3,4-dihydro-3-méthyl-2,2-dioxo-1,2-benzoxathiine-8-ylsulfonyl)-N'-(4,6-diméthyl-1-oxo-1,3,5-triazine-2-yl)-urée selon la revendication 1.

13. La N-(3,4-dihydro-3-méthyl-2,2-dioxo-1,2-benzoxathiine-8-ylsulfonyl)-N'-(2,6-diméthoxy-5-méthyl-pyrimidine-4-yl)-urée selon la revendication 1.

14. La N-(3,4-dihydro-3-méthyl-2,2-dioxo-1,2-benzoxathiine-8-ylsulfonyl)-N'-(5,6-diméthyl-1,2,4-triazine-3-yl)-urée selon la revendication 1.

15. Procédé de préparation des composés de formule I selon la revendication 1, caractérisé en ce que l'on fait réagir un sulfonamide de formule II

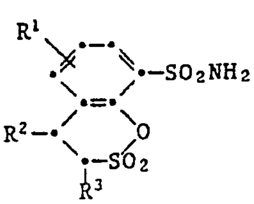

(II)

dans laquelle $R^1$, $R^2$ et $R^3$ ont les significations indiquées en référence à la formule I, en présence d'une base, avec un carbamate de formule III

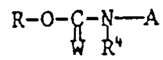 (III)

dans laquelle A, $R^4$ et W ont les significations indiquées en référence à la formule I et R représente un groupe phényle, alkyle ou phényle substitué, et le cas échéant on convertit en un sel.

16. Procédé de préparation des composés de formule I selon la revendication 1, caractérisé en ce que l'on fait réagir un sulfonylcarbamate de formule IV

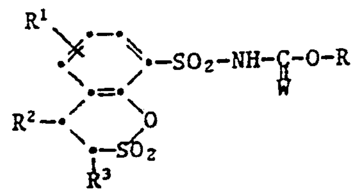 (IV)

dans laquelle $R^1$, $R^2$, $R^3$ et W ont les significations indiquées en référence à la formule I et R représente un groupe phényle, alkyle ou phényle substitué, avec une amine de formule V

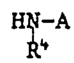 (V)

dans laquelle A et $R^4$ ont les significations indiquées en référence à la formule I, et le cas échéant on convertit en un sel.

17. Procédé de préparation des composés de formule I selon la revendication 1, caractérisé en ce que l'on fait réagir un sulfonylisocyanate de formule VI

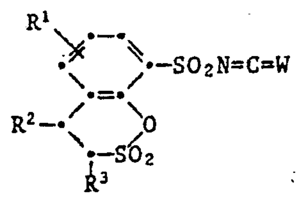 (VI)

dans laquelle $R^1$, $R^2$, $R^3$ et W ont les significations indiquées en référence à la formule I, avec une amine de formule V

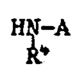 (V)

dans laquelle A et $R^4$ ont les significations indiquées en référence à la formule I, et le cas échéant on convertit en un sel.

18. Procédé de préparation des sels d'addition des composés de formule I selon l'une des revendications 15 à 17, caractérisé en ce que l'on fait réagir une sulfonylurée de formule I avec une amine, un hydroxyde de métal alcalin ou alcalino-terreux ou une base d'ammonium quaternaire.

19. Un produit herbicide et inhibiteur de la croissance des végétaux, caractérisé en ce qu'il contient en tant que substance active, avec des véhicules et/ou d'autres additifs, au moins une sulfonylurée substituée de formule I, revendication 1.

20. Procédé pour combattre les croissances de végétaux indésirables, caractérisé en ce que l'on applique sur les plantes ou leur biotope une substance active de formule I selon la revendication 1 ou un produit contenant cette substance active en quantité efficace.

21. Procédé pour inhiber la croissance des végétaux, caractérisé en ce que l'on applique sur les plantes ou leur biotope une substance active de formule I selon la revendication 1 ou un produit contenant cette substance active, en quantité efficace.

22. Procédé pour agir sur la croissance des végétaux dans le but d'augmenter le rendement, caractérisé en ce que l'on applique sur les plantes ou leur biotope une substance active de formule I selon la revendication 1 ou un produit contenant cette substance active, en quantité efficace.

23. Procédé selon la revendication 20, pour la lutte sélective en pré- ou post-levée contre les végétaux adventices dans les cultures de végétaux utiles.

24. Procédé selon la revendication 21, pour limiter la croissance des végétaux au stade deux feuilles, caractérisé en ce que l'on applique les substances actives en pré-levée.

25. Composés selon la revendication 1, caractérisés en ce que

$R^1$ représente l'hydrogène, un halogène, un groupe nitro, alkyle en $C_1$-$C_4$, halogénoalkyle en $C_1$-$C_4$, alcoxy en $C_1$-$C_4$, halogénoalcoxy en $C_1$-$C_4$, (alcoxy en $C_1$-$C_4$)-carbonyle, alkylthio en $C_1$-$C_4$, alkylsulfinyle en $C_1$-$C_4$, alkylsulfonyle en $C_1$-$C_4$ ou alcoxyalcoxy en $C_2$-$C_5$,

$R^2$ et $R^3$ représentent chacun, indépendamment l'un de l'autre, l'hydrogène ou un groupe alkyle en $C_1$-$C_4$,

$R^4$ représente l'hydrogène, un groupe méthyle ou éthyle,

W représente l'oxygène ou le soufre, et

A représente un groupe

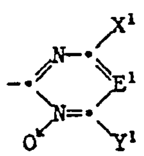

A1

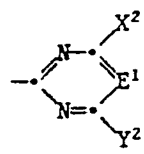

A2

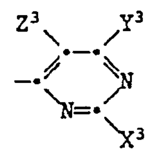

A3

,

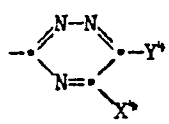

A4

ou

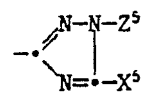

A5

,

dans lesquels

$X^1$, $X^2$, $X^3$, $X^4$, $X^5$ et $Y^1$ représentent chacun, indépendamment les uns des autres, un halogène, un groupe halogénoalcoxy en $C_1$-$C_4$, halogénoalkyle en $C_1$-$C_4$, alcoxy en $C_1$-$C_4$, alkyle en $C_1$-$C_4$, diméthylamino, méthylamino, éthylamino, amino ou alcoxyalkyle en $C_2$-$C_4$,

$E^1$ représente l'azote ou le pont méthine,

$Y^2$ représente un groupe cyclopropyle, diméthoxyméthyle, diéthoxyméthyle,

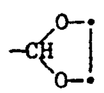

ou

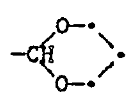

,

$Y^3$ représente un groupe méthyle, méthoxy, éthyle, éthoxy, halogénoalkyle en $C_1$-$C_2$ ou halogénoalcoxy en $C_1$-$C_2$,

$Y^4$ représente l'hydrogène, un groupe méthoxy, éthoxy, un halogène ou un groupe alkyle en $C_1$-$C_4$,

$Z^3$ représente l'hydrogène, un groupe méthyle, éthyle, méthoxy, éthoxy, méthoxycarbonyle, éthoxycarbonyle, un halogène, un groupe cyano, nitro méthylthio, méthylsulfinyle ou méthylsulfonyle,

$Z^3$ et $Y^3$ forment ensemble un pont alkylène en $C_2$-$C_4$ ou un pont alkylène en $C_2$-$C_4$ interrompu par l'oxygène, et

$Z^5$ représente un groupe méthyle ou éthyle,

et les sels de ces composés ; sous réserve que $R^3$ doit représenter un groupe alkyle en $C_1$-$C_4$ lorsque A représente l'un des groupes A2 ou A5.